(19) Europäisches Patentamt
European Patent Office
Office européen des brevets

(11) Veröffentlichungsnummer: **0 011 603**
**A1**

# (12) EUROPÄISCHE PATENTANMELDUNG

(21) Anmeldenummer: **79810145.7**

(22) Anmeldetag: **05.11.79**

(51) Int. Cl.³: **C 07 D 223/22**
**A 61 K 31/55**

(30) Priorität: **10.11.78 CH 11588 78**

(43) Veröffentlichungstag der Anmeldung: **28.05.80 Patentblatt 80/11**

(84) Benannte Vertragsstaaten: **AT BE CH DE FR GB IT LU NL SE**

(71) Anmelder: **CIBA-GEIGY AG**
**Patentabteilung Postfach**
**CH-4002 Basel(CH)**

(72) Erfinder: **Blattner, Hans**
**Burgstrasse 116**
**CH-4125 Riehen(CH)**

(72) Erfinder: **Storni, Angelo, Dr.**
**Im Feuerbusch 3**
**CH-4310 Rheinfelden(CH)**

(54) **Neues substituiertes 5H-Dibenz(b,f)azepin, Verfahren zu seiner Herstellung und pharmazeutische Präparate, welche diese Verbindung enthalten.**

(57) Das substituierte 5H-Dibenz[b,f]azepin der Formel

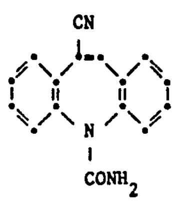

Verfahren zu seiner Herstellung, Arzneimittel, welche die neue Verbindung enthalten und ihre Verwendung zur Behandlung von Epilepsie.

EP 0 011 603 A1

CIBA-GEIGY AG,
Basel (Schweiz)

4-12109/-

Neues substituiertes 5H-Dibenz[b,f]azepin, Verfahren zu seiner Herstellung und pharmazeutische Präparate, welche diese Verbindung enthalten.

Die Erfindung betrifft ein neues substituiertes 5H-Dibenz[b,f]azepin, Verfahren zu seiner Herstellung und pharmazeutische Präparate, welche diese Verbindung enthalten.

Das erfindungsgemässe neue substituierte 5H-Dibenz[b,f]azepin entspricht der Formel I

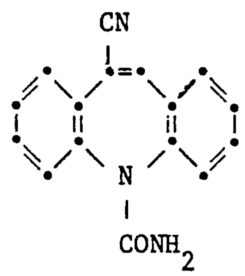

(I)

Die neue Verbindung besitzt wertvolle pharmakologische Eigenschaften, insbesondere eine hohe antikonvulsive Wirksamkeit. Diese Wirkungsqualität zeigt sich in Standard-Testverfahren für Antikonvulsiva, besonders im Strychnin- und im Picrotoxin-Antagonismus an der Maus, im Dosisbereich von etwa 30 bis etwa 100 mg/kg per os bzw. etwa 60 bis etwa 100 mg/kg per os. Diese Tests wurden wie folgt durchgeführt:

Antagonismus gegen durch Picrotoxin induzierte Krämpfe:

Durch intraperitoneale Injektionen von 7,5 mg/kg Picrotoxin werden an Mäusen klonische Krämpfe ausgelöst. Die Schutzwirkung der einer Stunde vorher oral verabreichten Test-Substanz wird registriert. Pro Dosis werden 10 Versuchstiere verwendet; eine Gruppe dient als

Kontrolle. Die $ED_{50}$, d.h. die Dosis, bei welcher 50% der Tiere geschützt werden, wird graphisch bestimmt.

Antagonismus gegen durch Strychnin induzierte tödliche Krämpfe

Durch intraperitoneale Injektion von 2,5 mg/kg Strychninnitrat werden an Mäusen tödliche Krämpfe ausgelöst. Eine Stunde vorher wird die Test-Substanz oral verabreicht. Pro Dosis werden 10 Versuchstiere verwendet; eine Gruppe dient als Kontrolle. Die $ED_{50}$, d.h. die Dosis, bei welcher 50% der Tiere mindestens 10 Minuten überleben, wird graphisch bestimmt.

Ebenfalls wirksam ist die Verbindung der Formel I im Elektroschock-Test an der Maus und an der Ratte bei oraler Verabreichung von Dosen von 6-30 mg/kg.Weiter zeichnet sich die Verbindung der Formel I durch niedrige Toxizität aus, ebenfalls gering sind im Vergleich zur starken antikonvulsiven Wirksamkeit Nebenwirkungen, wie Sedation und muskuläre Hypotonie.

0011603



Diese Verbindung der Formel I kann daher zur Behandlung der Epilepsie verwendet werden. Ferner kann die neue Verbindung als Ausgansstoff- oder Zwischenprodukt für die Herstellung anderer, insbesondere therapeutisch wirksamer Verbindungen dienen.

Die Verbindung wird nach an sich bekannten Methoden erhalten.

So kann man eine Verbindung der Formel

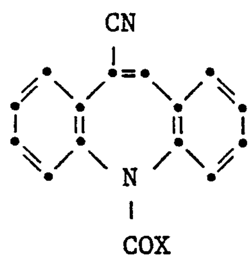

(II)

in welcher X Halogen mit einer Atomnummer von mindestens 17 bedeutet, mit Ammoniak oder Ammoniak-abgebenden Mitteln umsetzen.

Halogen ist demgemäss Brom oder Jod, aber insbesondere Chlor.

Die Umsetzung wird vorzugsweise in einem organischen Lösungsmittel, z.B. in einem niederen Alkanol, wie Aethanol, Isopropanol oder Butanol, in einer ätherartigen Flüssigkeit, wie Tetrahydrofuran oder Dioxan, oder einem Kohlenwasserstoff, wie Benzol oder Toluol, bei Raumtemperatur oder vorzugsweise in der Wärme, z.B. bei der Siedetemperatur des verwendeten Lösungsmittels, durchgeführt. Das benötigte Ammoniak kann als Gas am Anfang oder während der ganzen Reaktionsdauer eingeleitet, oder bei Verwendung eines

mit Wasser mischbaren Lösungsmittels auch als konzentrierte wässrige Lösung eingesetzt werden. Man kann aber auch flüssiges Ammoniak verwenden und die Umsetzung nötigenfalls im geschlossenen Gefäss durchführen. Als Ammoniak-abgebendes Mittel eignet sich insbesondere Hexamethylentetramin.

Ausgangsstoffe der Formel II können z.B. erhalten werden durch Umsetzen von gegebenenfalls in 5-Stellung acyliertem 10-Brom-5H-dibenz[b,f]azepin mit Kupfer(I)-cyanid und anschliessender Bildung des entsprechenden Carbonylhalogenids durch Umsetzung des, gegebenenfalls unter Abspaltung des 5-Acylrestes, entstandenen Cyan-5H-dibenz[b,f]azepins mit einem Dihalogenid der Kohlensäure, z.B. Phosgen.

Die neue Verbindung der Formel I kann z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln,welche den Wirkstoff zusammen mit Verdünnungsmitteln, z.B. Laktose, Dextrose, Saccharose, Mannitol, Sorbitol, Cellulose und/oder Glycin, und/oder Schmiermittel, z.B. Kieselerde, Talk, Stearinsäure oder Salze davon, wie Magnesium- oder Cacliumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke,

Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel aufweisen. Ferner kann man die neue Verbindung der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegende pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die Erfindung betrifft ebenfalls die Verwendung der Verbindung der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 1 und etwa 20 mg/kg und für Warmblüter mit einem Gewicht von etwa 70 kg vorzugsweise zwischen etwa 100 mg und etwa 800 mg.

Die folgenden Beispiele dienen zur Illustration der Erfindung; Temperaturen werden in Celsiusgraden angegeben.

Beispiel 1: 21,8 g (0,1 Mol) 10-Cyan-5H-dibenz[b,f]azepin werden unter Rühren in eine Lösung von 19,8 g Phosgen (0,2 Mol) 700 ml absolutem Toluol eingetragen. Das Gemisch wird 30 Stunden bei einer Innentemperatur von 50-53° gerührt. Hierauf dampft man das Reaktionsgemisch im Rotationsverdampfer vollständig ein, wobei das rohe 10-Cyan-5H-dibenz[b,f]azepin-5-carbonylchlorid von Smp. 148-153° zurückbleibt.

Das erhaltene Rohprodukt wird unter Rühren bei 70° in 600 ml absolutem Aethanol gelöst. In diese Lösung leitet man während 2 Stunden Ammoniakgas ein, wobei die Reaktionslösung immer unter Rückfluss gehalten wird. Anschliessend dampft man das Reaktionsgemisch im Rotationsverdampfer ein, wäscht den Rückstand mit Wasser und kristallisiert das 10-Cyan-5H-dibenz[b,f,]azepin-5-carboxamid nach dem Trocknen aus Toluol um, Smp. 212-215°.

Der Ausgangsstoff kann wie folgt hergestellt werden:

27,2 g (0,1 Mol) 10-Brom-5H-dibenz[b,f,]azepin, 10,7 g (0,12 Mol) Kupfer(I)-cyanid und 50 ml Dimethyformamid werden unter Rühren bei einer Badtemperatur von 150° 1 1/2 Stunden erhitzt. Anschliessend wird das Reaktionsgemisch auf 40° abgekühlt und mit 200 ml einer 50%-igen wässerigen Aethylendiaminlösung und 200 ml Methylenchlorid 2 Stunden stark gerührt. Dann wird die organische Phase abgetrennt und die wässerige Phase noch zweimal mit 100 ml

Methylenchlorid extrahiert. Die vereinigten organischen Lösungen werden mit Wasser gewaschen, über Natriumsulfat getrocknet und eingedampft. Der Rückstand, das 10-Cyan-5H-dibenz[b,f]azepin, wird aus Aethanol umkristallisiert und schmilzt bei 143-145°.

Beispiel 2: Tabletten, enthaltend je 50 mg 10-Cyan-5H-dibenz[b,f]azepin -5-carboxamid, können wie folgt hergestellt werden:

Zusammensetzung (10000 Tabletten)

| | |
|---|---|
| 10-Cyan-5H-dibenz[b,f]azepin-5-carboxamid | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Aethanol | q.s. |

Das 10-Cyan-5H-dibenz[b,f]azepin-5-carboxamid wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 3: Lacktabletten, enthaltend je 100 mg 10- Cyan-5H-dibenz [b,f]azepin-5-carboxamid, können wie folgt hergestellt werden:

Zusammensetzung (für 1000 Tabletten)

| | |
|---|---|
| 10-Cyan-5H-dibenz[b,f]azepin-5-carboxamid | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 |
| Schellack | 0.64 g |
| Wasser | q.s. |
| Methylenchlorid | q.s. |

Das 10-Cyan-5H-dibenz[b,f]azepin-5-carboxamid, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und das Schellacks in Methylenchlorid lackiert; Endgewicht der Lacktablette: 283 mg.

Patentansprüche

1.) Das 10-Cyan-5H-dibenz]b,f]azepin-5-carboxamid der Formel

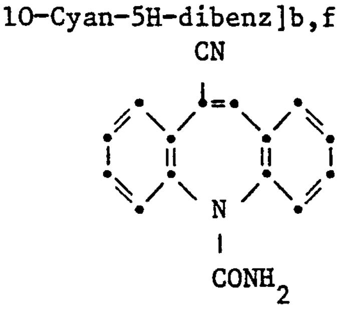

2.) Verfahren zur Herstellung des 10-Cyan-5H-dibenz[b,f]azepin-5-carboxamids, dadurch gekennzeichnet, dass man eine Verbindung der Formel

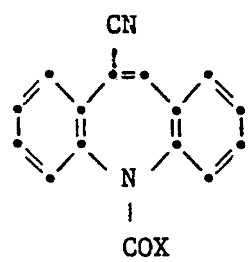

(II)

in welcher X Halogen mit einer Atomnummer von mindestens 17 bedeutet, mit Ammoniak oder Ammoniak-abgebenden Mitteln umsetzt.

3.) Pharmazeutische Zusammensetzungen, gekennzeichnet durch einen Gehalt an der Verbindung gemäss Anspruch 1 und mindestens einen pharmazeutischen Trägerstoff.

4.) Das 10-Cyano-5H-dibenz[b,f]azepin-5-carboxamid zur Anwendung in einem Verfahren zur therapeutischen Behandlung des menschlichen oder tierischen Körpers.

5.) Das 10-Cyano-5H-dibenz[b,f]azepin-5-carboxamid zur Verwendung bei der Behandlung der Epilepsie.

0011603

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

Nummer der Anmeldung
EP 79 810 145.7

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.3) |
|---|---|---|---|
| | DE - B - 1 136 707 (J.R. GEIGY)<br>* Anspruch *<br>--<br>DE - A1 - 2 542 335 (CIBA-GEIGY)<br>* Anspruch 9 *<br>---- | 2 | C 07 D 223/22<br>A 61 K 31/55 |

RECHERCHIERTE SACHGEBIETE (Int. Cl.3)

A 61 K 31/55
C 07 D 223/22

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3
Unvollständig recherchierte Patentansprüche:
Nicht recherchierte Patentansprüche: 4,5
Grund für die Beschränkung der Recherche:

Verfahren zur therapeutischen Behandlung des menschlichen oder tierischen Körpers

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 29-01-1980 | FROELICH |

EPA Form 1505.1 06.78